Europäisches Patentamt

European Patent Office

Office européen des brevets

(11) Veröffentlichungsnummer: **0 516 020 A2**

# EUROPÄISCHE PATENTANMELDUNG

(21) Anmeldenummer: **92108790.4**

(22) Anmeldetag: **25.05.92**

(51) Int. Cl.5: **C07C 17/42**, C07C 19/08

(30) Priorität: **28.05.91 DE 4117379**

(43) Veröffentlichungstag der Anmeldung:
**02.12.92 Patentblatt 92/49**

(84) Benannte Vertragsstaaten:
**AT BE CH DE DK ES FR GB GR IT LI NL SE**

(71) Anmelder: **HOECHST AKTIENGESELLSCHAFT**
**Postfach 80 03 20**
**W-6230 Frankfurt am Main 80(DE)**

(72) Erfinder: **Schütz, Claudia**
**Dresdner Strasse 9**
**W-6093 Flörsheim am Main(DE)**
Erfinder: **Behme, Klaus-Jürgen**
**Kurmainzer Strasse 25**
**W-6239 Eppstein/Taunus(DE)**
Erfinder: **Deger, Hans-Matthias, Dr.**
**Herrnpfad 10**
**W-6238 Hofheim am Taunus(DE)**

(54) **Stabilisierung von Dichlortrifluorethan enthaltenden Mischungen.**

(57) Die Erfindung betrifft eine Mischung, die im wesentlichen aus 2,4-Diphenyl-4-methyl-penten, 4-tert.-Butylbrenzkatechin und mindestens einem der beiden Dichlortrifluorethane $CF_3$-$CHCl_2$ und $CClF_2$-$CHClF$ besteht, sowie ein Verfahren zur Stabilisierung von Mischungen, die mindestens eines der Dichlortrifluorethane $CF_3$-$CHCl_2$ und $CClF_2$-$CHClF$ sowie mindestens ein Aminopolyol und/oder Amin enthalten, dadurch, daß man den Mischungen eine wirksame Menge 2,4-Diphenyl-4-methylpenten und 4-tert.-Butylbrenzkatechin zusetzt.

EP 0 516 020 A2

Die Erfindung betrifft die Stabilisierung Von Dichlortrifluorethan enthaltenden Mischungen, die zur Herstellung von Polyurethan-Schaum verwendet werden.

Dichlortrifluorethan ($CF_3$-$CHCl_2$ und $CClF_2$-$CHClF$) ist vorgesehen als Ersatz für das wegen der vermuteten Schädigung der atmosphärischen Ozonschicht bald nicht mehr zur Verfügung stehende Trichlorfluormethan (FCKW 11), welches Verwendung findet als Dämmgas und Treibmittel bei der Herstellung von Schaumstoffen auf der Basis Polyisocyanaten, insbesondere von Polyurethan- und Polyisocyanuratschaumstoffen (US-PS 3 183 192). Die Herstellung solcher Schaumstoffe ist bekannt und beispielsweise im Kunststoff-Handbuch, Band VII, Polyurethane, Carl Hanser Verlag München, Wien (1983), Seiten 246 bis 331, sowie in EP-A-0 077 964, EP-A-0 334 059, DE-AS 1 694 138 (= GB-PS 1 209 243) beschrieben.

In Ullmanns Enzyklopädie der technischen Chemie (1980), Band 19, Seiten 301 bis 341, sind die verwendbaren Rohstoffe und die möglichen Verfahren zur Herstellung von Polyurethanhartschaumstoffen zusammenfassend beschrieben.

Des weiteren sind entsprechende Hinweise in Kirk-Othmer, Encycl. of Chem. Technology, 3rd Edition, Vol. 11 (1980), Seiten 87 - 89 und Vol. 23 (1983), Seiten 576 - 607 zu finden.

Zur Herstellung von Polyurethanschaumstoffen wird in vielen Fällen bereits eine Mischung aus Polyolen, Katalysatoren, Schaumstabilisatoren, Dichlortrifluorethan als Treibmittel, Wasser sowie gegebenenfalls flammhemmenden Mitteln und sonstigen Zusatzstoffen (diese Mischung wird im folgenden auch als A-Komponente bezeichnet) beim Verwender angeliefert. In diesem Fall ist das Dichlortrifluorethan mit den sonstigen Bestandteilen der A-Komponente längere Zeit in Kontakt, bevor die Umsetzung mit Polyisocyanat (im folgenden auch als B-Komponente bezeichnet) zu Polyurethan-Schaumstoff erfolgt. Eine Alternative ist die Herstellung der A-Komponente aus den vorgenannten Einzelkomponenten oder aus Teilmischungen von diesen direkt beim Verschäumer. Auch in diesem Fall muß die A-Komponente bis zur Verschäumung gelagert werden.

Für die Verschäumung geeignete Polyisocyanate sind aliphatische, cycloaliphatische und aromatische Di- oder Polyisocyanate. Bevorzugt sind 2,4- und 2,6-Toluyldiisocyanat, Diphenylmethandiisocyanat, Polymethylenpolyphenylisocyanat und Mischungen davon. Es können auch Carbodiimidgruppen, Urethangruppen, Allophanatgruppen, Isocyanuratgruppen, Harnstoffgruppen oder Biuretgruppen enthaltende Polyisocyanate, die man als "modifizierte Polyisocyanate" oder "Isocyanat-Präpolymere" bezeichnet, verwendet werden.

Als Polyole werden in der Regel Verbindungen eingesetzt, die mindestens zwei gegenüber Isocyanatgruppen reaktionsfähige Wasserstoffatome enthalten, beispielsweise Verbindungen auf Polyether-, Polyester- und Aminbasis, sowie Amino- und/oder Carboxyl- und/oder Thiolgruppen aufweisende Verbindungen mit im allgemeinen 2 - 8 gegenüber Polyisocyanaten reaktionsfähigen Wasserstoffatomen (US-PS 3 183 192). Dabei werden oft Hydroxylgruppen enthaltende Aminoverbindungen (Aminopolyole) eingesetzt, entweder allein oder neben aminogruppenfreien Polyolen.

Zur Umsetzung der Polyole und/oder Aminopolyole mit den Polyisocyanaten wird ein Katalysator zugegeben. Als Katalysatoren werden üblicherweise Aminoverbindungen, insbesondere tertiäre (z.B. Dimethylcyclohexylamin) eingesetzt. Diese Aminoverbindungen werden der vorgenannten A-Komponente zugegeben. Es können aber auch aminogruppenfreie Verbindungen als Katalysatoren eingesetzt werden, wie z.B. organische Zinnverbindungen, etwa Dibutylzinndilaurat.

Es hat sich jedoch gezeigt, daß die katalytisch wirksame Aminoverbindungen und/oder Aminopolyole enthaltenden Mischungen mit den Wasserstoffatome enthaltenden Dichlortrifluorethanen H-FCKW 123 ($CF_3$-$CHCl_2$) und H-FCKW 123 a ($CClF_2$-$CHClF$) unter Abspaltung von Chlorwasserstoff zu wasserstoffreicheren Chlorhydrofluoralkanen, insbesondere zu H-FCKW 133a ($CF_3$-$CH_2Cl$), reagieren. Das Ausmaß dieser Reaktion hängt ab von verschiedenen Parametern, wie pH-Wert, Temperatur und Metallionengehalt. Der gebildete Chlorwasserstoff stört die anschließende Verschäumungsreaktion mit dem Polyisocyanat erheblich. Mitunter treten unter den genannten Bedingungen auch kristalline Ausscheidungen in der A-Komponente auf, welche die Dosierung mittels Präzisionsdosierpumpen stören. H-FCKW 133a ist aufgrund seiner kanzerogenen Eigenschaften (in Tierversuchen) als Abbauprodukt unerwünscht.

Es ist zwar prinzipiell möglich, unter Verwendung hochreaktiver Polyole mit aminfreien Formulierungen zu arbeiten, bei denen eine Dechlorierungsreaktion normalerweise nicht auftritt. Als Katalysatoren können hierbei metallorganische Verbindungen, wie z.B. Dibutylzinndilaurat, verwendet werden.

Aufgabe der vorliegenden Erfindung ist es, auch den Einsatz von Aminopolyole und/oder andere Aminoverbindungen enthaltenden Systemen unter Verwendung von Dichlortrifluorethan zu ermöglichen, ohne daß eine Bildung von HCl oder H-FCKW 133a eintritt.

Aus der DE-PS 1 207 626 (US-PS 3 183 192) ist bekannt, daß ungesättigte Verbindungen wie Butadien, Isopren, Styrol, alpha-Methylstyrol oder 1-Alkene mit 4 bis 18 Kohlenstoffatomen als Stabilisatoren für die (vollhalogenierten) Chlorfluoralkane Trichlorfluormethan und Trichlortrifluorethan in solchen verschäumbaren

2

Massen eingesetzt werden können. Aus der DE-PS 3 139 401 (US-PS 4 463 189) ist bekannt, daß auch 2,4-Diphenyl-4-methyl-penten (DMP) die unerwünschte Reaktion von Aminopolyolen mit diesen Chlorfluoralkanen unterdrückt.

Es wurde gefunden, daß DMP auch die Dechlorierungsreaktionen der (H-Atome enthaltenden) Dichlortrifluorethane in gewissem Umfang unterdrückt. Weiterhin wurde gefunden, daß 4-tert.-Butylbrenzkatechin diese Dechlorierungsreaktionen ebenfalls in gewissem Umfang verhindert.

Überraschenderweise wurde dann gefunden, daß Mischungen aus 4-tert.-Butylbrenzkatechin und DMP diese Dechlorierungsreaktion vollständig unterdrücken, so daß insbesondere die Bildung von H-FCKW 133a ($CF_3$-$CH_2Cl$) und HCl unterbleibt.

Ein Gegenstand der Erfindung ist eine Mischung, die im wesentlichen aus DMP, 4-tert.-Butylbrenzkatechin und mindestens einem der beiden Dichlortrifluorethane $CF_3$-$CHCl_2$ und $CClF_2$-CHClF besteht. Im allgemeinen enthält die Mischung eine solche Menge an DMP und 4-tert.-Butylbrenzkatechin, die ausreicht zur Unterdrückung der genannten Dechlorierungsreaktion der Dichlortrifluorethane, wenn diese zusammen mit Aminopolyolen und/oder anderen Aminoverbindungen bei der Polyurethan-Verschäumung eingesetzt werden, wobei aber der Gehalt an DMP und 4-tert.-Butylbrenzkatechin normalerweise höchstens 10 Gew.-% beträgt. Vorzugsweise enthält die Mischung 0,1 bis 5 Gew.-% DMP und 0,02 bis 2 Gew.-% 4-tert.-Butylbrenzkatechin, insbesondere 0,2 bis 2 Gew.-% DMP und 0,05 bis 1 Gew.-% 4-tert.-Butylbrenzkatechin, bezogen auf die Gesamtmenge an Dichlortrifluorethanen.

Weiterhin ist Gegenstand der Erfindung ein Verfahren zur Stabilisierung von Mischungen, die mindestens eines der Dichlortrifluorethane $CF_3$-$CHCl_2$ und $CClF_2$-CHClF sowie Aminopolyole und/oder primäre, sekundäre oder tertiäre Amine enthalten, das dadurch gekennzeichnet, ist, daß man den Mischungen eine wirksame Menge 2,4-Diphenyl-4-methylpenten und 4-tert.-Butylbrenzkatechin zusetzt. Die bevorzugten und die besonders bevorzugten Mengen an DMP und 4-tert.-Butylbrenzkatechin sind dieselben wie bei der eben erwähnten Mischung.

Die Mischungen aus 4-tert.-Butylbrenzkatechin und DMP sind in Dichlortrifluorethan löslich und beeinflussen nicht die Verschäumungsreaktion.

DMP ist unter dem Namen "Dimeres alpha-Methylstyrol" (GAS-Nr. 6144-04-3) ein handelsübliches Produkt und kann z.B. nach US-PS 2 429 719 hergestellt werden.

Es besteht im allgemeinen aus den beiden Isomeren 2,4-Diphenyl-4-methyl-penten-(1) und 2,4-Diphenyl-4-methyl-penten-(2). Das Isomerenverhältnis Penten-(1)/Penten-(2) beträgt dabei im allgemeinen etwa 1:1 bis 9:1.

Anstelle des Gemisches der beiden Pentene läßt sich auch ein technisches Gemisch einsetzen, das neben diesen beiden Isomeren noch einen geringe Anteil an 1,1,3-Trimethyl-3-phenylindan enthält. Ein solches Gemisch ist z.B. nach dem Verfahren der US-PS 2 429 719 erhältlich und enthält im allgemeinen 45 bis 89 Gew.-% 2,4-Diphenyl-4-methyl-pentan-(1), 10 bis 45 Gew.-% 2,4-Diphenyl-4-methyl-penten-(2) und 1 bis 20 Gew.-% 1,1,3-Trimethyl-3-phenyl-indan. Bei Einsatz eines solchen technischen Gemischs muß natürlich eine solche Menge gewählt werden, daß die oben angegebenen Prozentgehalte an reinem DMP erreicht werden.

Natürlich kann man auch die reinen Isomeren einzeln einsetzen, aber eine Trennung des bei der Herstellung des DMP entstehenden Isomerengemisches wäre ein unnötiger Aufwand.

4-tert.-Butylbrenzkatechin (mit der CAS-Nr. 98-29-3) ist bekannt als Polymerisationsinhibitor für monomere Olefine und ist eine handelsübliche Verbindung (Beilstein E III 6: 4671):

Prinzipiell können auch andere Chlorhydrofluoralkane, wie Chlortetrafluorethan, 1,1-Dichlor-1-fluorethan oder 1-Chlor-1,1-difluorethan, die als Treibmittel und Dämmgase für dieselben Anwendungen wie die Dichlortrifluorethane zur Aufschäumung von Polyurethanen geeignet sind, in gleicher Weise stabilisiert werden.

Die Erfindung wird durch die folgenden Beispiele näher erläutert. Dabei wird 4-tert.-Butylbrenzkatechin in den Tabellen als "But" abgekürzt.

Beispiele

Versuchsdurchführung:

Zur Durchführung der Prüfungen wurden jeweils 50 g der in den nachfolgenden Beispielen beschriebenen Mischungen in Aerosol-Glasflaschen eingewogen, diese sofort gasdicht verschlossen und bei einer Temperaturvon 50°C gelagert. Obwohl die Spaltprodukte (Chlorwasserstoff und $CF_3$-$CH_2Cl$) bereits bei Lagertemperaturen von ca. 20°C auftreten, wurde zur beschleunigten Prüfung die Temperatur von 50°C gewählt, da bekanntlich durch eine Temperaturerhöhung um 30°C chemische Reaktionen um ein Mehrfaches beschleunigt werden. Die Gemische wurden nach der jeweils angegebenen Zeit nach ihrem optischen Aussehen beurteilt und auf die Spaltprodukte analysiert.

Die Mengen der Spaltprodukte sind in Gew.-%, bezogen auf die gesamte eingewogene Mischung angegeben, wobei Chlorwasserstoff als Chlorid bestimmt wurde.

Als tertiäre Aminoverbindungen, die bei einer Umsetzung mit Polyisocyanat als Katalysatoren wirken würden, sind Dimethylcyclohexylamin und Dimethylaminoethanol untersucht worden.

Beispiel 1:

a) Es wurden 70 Gew.-% Dimethylcyclohexylamin (DMCHA) und 30 Gew.-% $CHCl_2$-$CF_3$ vermischt und 14 Tage bei 50°C gelagert. Die Ergebnisse der Analyse sind in Tabelle 1 dargestellt.

b) Es wurde verfahren wie in Beispiel 1a), jedoch wurde statt $CHCl_2$-$CF_3$ eine Mischung aus 93 Gew.-% $CHCl_2$-$CF_3$ und 7 Gew.-% $CClF_2$-$CHClF$ eingesetzt. Die Ergebnisse der Analyse sind in Tabelle 2 dargestellt.

c) Es wurde verfahren wie in Beispiel 1a), aber mit zusätzlich 1 Gew.-% DMP, bezogen auf den Gehalt an $CHCl_2$-$CF_3$. Die Ergebnisse sind in Tabelle 1 dargestellt.

d) Es wurde verfahren wie in Beispiel 1b), aber mit zusätzlich 1 Gew.-% DMP, bezogen auf die Gesamtmenge der Dichlortrifluorethan-Mischung aus 93 Gew.-% $CHCl_2$-$CF_3$ und 7 Gew.-% $CClF_2$-$CHClF$. Die Ergebnisse sind in Tabelle 2 dargestellt.

e) Es wurde verfahren wie in Beispiel 1a), aber mit zusätzlich 1 Gew.-% 4-tert.-Butylbrenzkatechin, bezogen auf den Gehalt an $CHCl_2$-$CF_3$. Die Ergebnisse sind in Tabelle 1 dargestellt.

f) Es wurde verfahren wie in Beispiel 1b), aber mit zusätzlich 1 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-$CHClF$. Die Ergebnisse sind in Tabelle 2 dargestellt.

g) Es wurde verfahren wie in Beispiel 1a), aber mit zusätzlich 1 Gew.-% einer Stabilisatormischung, bestehend aus 50 Gew.-% DMP und 50 Gew.-% 4-tert.-Butylbrenzkatechin, bezogen auf den Gehalt an $CHCl_2$-$CF_3$. Die Ergebnisse sind in Tabelle 1 dargestellt.

h) Es wurde verfahren wie in Beispiel 1b), aber mit zusätzlich 1 Gew.-% einer Stabilisatormischung, bestehend aus 50 Gew.-% DMP und 50 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-$CHClF$. Die Ergebnisse sind in Tabelle 2 dargestellt.

Beispiel 2:

a) Untersucht wurde eine Mischung von 70 Gew.-% Dimethylaminoethanol (DMEA) mit 30 Gew.-% $CHCl_2$-$CF_3$. Die Ergebnisse sind in Tabelle 3 dargestellt.

b) Es wurde verfahren wie in Beispiel 2a), jedoch wurde statt $CHCl_2$-$CF_3$ eine Mischung aus 93 Gew.-% $CHCl_2$-$CF_3$ und 7 Gew.-% $CClF_2$-$CHClF$ eingesetzt. Die Ergebnisse der Analyse sind in Tabelle 4 dargestellt.

c) Es wurde verfahren wie in Beispiel 2a), aber mit zusätzlich 1 Gew.-% DMP, bezogen auf den Gehalt an $CHCl_2$-$CF_3$. Die Ergebnisse sind in Tabelle 3 dargestellt.

d) Es wurde verfahren wie in Beispiel 2b), aber mit zusätzlich 1 Gew.-% DMP, bezogen auf die Gesamtmenge der Dichlortrifluorethan-Mischung aus 93 Gew.-% $CHCl_2$-$CF_3$ und 7 Gew.-% $CClF_2$-$CHClF$. Die Ergebnisse sind in Tabelle 4 dargestellt.

e) Es wurde verfahren wie in Beispiel 2a), aber mit zusätzlich 1 Gew.-% 4-tert.-Butylbrenzkatechin, bezogen auf den Gehalt an $CHCl_2$-$CF_3$. Die Ergebnisse sind in Tabelle 3 dargestellt.

f) Es wurde verfahren wie in Beispiel 2b), aber mit zusätzlich 1 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-$CHClF$. Die Ergebnisse sind in Tabelle 4 dargestellt.

g) Es wurde verfahren wie in Beispiel 2a), aber mit zusätzlich 1 Gew.-% einer Stabilisatormischung, bestehend aus 50 Gew.-% DMP und 50 Gew.-% 4-tert.-Butylbrenzkatechin, bezogen auf den Gehalt an $CHCl_2$-$CF_3$. Die Ergebnisse sind in Tabelle 3 dargestellt.

h) Es wurde verfahren wie in Beispiel 2b), aber mit zusätzlich 1 Gew.-% einer Stabilisatormischung, bestehend aus 50 Gew.-% DMP und 50 Gew.-% 4-tert.-Butylbrenzkatechin, bezogen auf die Gesamtmenge der Dichlortrifuorethan-Mischung aus 93 Gew.-% $CHCl_2$-$CF_3$ und 7 Gew.-% $CClF_2$-$CHClF$. Die Ergebnisse sind in Tabelle 4 dargestellt.

Beispiel 3:

a) Nach 1 Tag, nach 7 Tagen und nach 14 Tagen wurde eine Mischung aus 70 Gew.-% Aminopolyol auf Basis Ethylendiamin und Propylenoxid der Hydroxylzahl 450 und 30 Gew.-% $CHCl_2$-$CF_3$ untersucht. Die Ergebnisse sind in Tabelle 5 dargestellt.

b) Es wurde verfahren wie in Beispiel 3a), jedoch wurde statt $CHCl_2$-$CF_3$ eine Mischung aus 93 Gew.-% $CHCl_2$-$CF_3$ und 7 Gew.-% $CClF_2$-CHClF eingesetzt. Die Ergebnisse sind in Tabelle 6 dargestellt.

c) Es wurde verfahren wie in Beispiel 3a), aber mit zusätzlich 1 Gew.-% DMP, bezogen auf den Gehalt an $CHCl_2$-$CF_3$. Die Ergebnisse nach 14 Tagen sind in Tabelle 5 dargestellt.

d) Es wurde verfahren wie in Beispiel 3b), aber mit zusätzlich 1 Gew.-% DMP, bezogen auf die Gesamtmenge der Dichlortrifluorethan-Mischung aus 93 Gew.-% $CHCl_2$-$CF_3$ und 7 Gew.-% $CClF_2$-CHClF. Die Ergebnisse der Analysen nach 14 Tagen sind in Tabelle 6 dargestellt.

e) Es wurde verfahren wie in Beispiel 3c), aber mit 1 Gew.-% 4-tert.-Butylbrenzkatechin, bezogen auf den Gehalt an $CHCl_2$-$CF_3$. Die Ergebnisse sind in Tabelle 5 dargestellt.

f) Es wurde verfahren wie in Beispiel 3d), aber mit 1 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-CHClF. Die Ergebnisse sind in Tabelle 6 dargestellt.

g) Es wurde verfahren wie in Beispiel 3c), aber mit 1 Gew.-% einer Stabilisatormischung, bestehend aus 50 Gew.-% DMP und 50 Gew.-% 4-tert.-Butylbrenzkatechin, bezogen auf den Gehalt an Dichlortrifluorethan. Die Ergebnisse sind in Tabelle 5 dargestellt.

h) Es wurde verfahren wie in Beispiel 3d), aber mit 1 Gew.-% einer Stabilisatormischung, bestehend aus 50 Gew.-% DMP und 50 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-CHClF. Die Ergebnisse sind in Tabelle 6 dargestellt.

Beispiel 4:

a) Untersucht wurde eine Mischung aus 73 Gew.-% des in Beispiel 3 verwendeten Aminopolyols und 1,5 Gew.-% DMCHA sowie 25,5 Gew.-% $CHCl_2$-$CF_3$. Die Ergebnisse sind in Tabelle 7 dargestellt.

b) Es wurde verfahren wie in Beispiel 4a), jedoch wurde statt $CHCl_2$-$CF_3$ eine Mischung aus 93 Gew.-% $CHCl_2$-$CF_3$ und 7 Gew.-% $CClF_2$-CHClF eingesetzt. Die Ergebnisse sind in Tabelle 8 dargestellt.

c) Es wurde verfahren wie in Beispiel 4a), aber mit zusätzlich 1 Gew.-% DMP, bezogen auf den Gehalt an $CHCl_2$-$CF_3$. Die Ergebnisse sind in Tabelle 7 dargestellt.

d) Es wurde verfahren wie in Beispiel 4b), aber mit zusätzlich 1 Gew.-% DMP, bezogen auf die Gesamtmenge der Dichlortrifluorethan-Mischung aus 93 Gew.-% $CHCl_2$-$CF_3$ und 7 Gew.-% $CClF_2$-CHClF. Die Ergebnisse sind in Tabelle 8 dargestellt.

e) Es wurde verfahren wie in Beispiel 4a), aber mit zusätzlich 1 Gew.-% 4-tert.-Butylbrenzkatechin, bezogen auf den Gehalt an $CHCl_2$-$CF_3$. Die Ergebnisse sind in Tabelle 7 dargestellt.

f) Es wurde verfahren wie in Beispiel 4b), aber mit zusätzlich 1 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-CHClF. Die Ergebnisse sind in Tabelle 8 dargestellt.

g) Es wurde verfahren wie in Beispiel 4a), aber mit zusätzlich 1 Gew.-% einer Stabilisatormischung, bestehend aus 50 Gew.-% DMP und 50 Gew.-% 4-tert.-Butylbrenzkatechin, bezogen auf den Gehalt an $CHCl_2$-$CF_3$. Die Ergebnisse der Analysen nach 14 und 26 Tagen sind in Tabelle 7 dargestellt.

h) Es wurde verfahren wie in Beispiel 4b), aber mit zusätzlich 1 Gew.-% einer Stabilisatormischung, bestehend aus 50 Gew.-% DMP und 50 Gew.-% 4-tert.-Butylbrenzkatechin, bezogen auf die Gesamtmenge der Dichlortrifluorethan-Mischung aus 93 Gew.-% $CHCl_2$-$CF_3$ und 7 Gew.-% $CClF_2$-CHClF. Die Ergebnisse der Analysen nach 14 und 26 Tagen sind in Tabelle 8 dargestellt.

Beispiel 5:

Die in Beispiel 4a) beschriebene Mischung wurde mit verschiedenen Anteilen an DMP und 4-tert.-Butylbrenzkatechin versetzt und wie in den vorgenannten Beispielen gelagert. Die Anteile der Stabilisatoren und die Ergebnisse sind in Tabelle 9 dargestellt.

Tabelle 1

| Beispiel | Lagerzeit (Tage) | Stabilisator (1 %, bezogen auf $CHCl_2$-$CF_3$) | Spaltprodukte | |
|---|---|---|---|---|
| | | | % $C_2H_2ClF_3$ | % $Cl^-$ |
| 1a | 14 | - | 0,13 | 0,04 |
| 1c | 14 | DMP | 0,10 | 0,03 |
| 1e | 14 | But | 0,05 | n.g. |
| 1g | 14 | DMP/But 1:1 | n.n. | n.n. |

Tabelle 2

| Beispiel | Lagerzeit (Tage) | Stabilisator (1 %, bezogen auf $CHCl_2$-$CF_3$/ $CCl_2$F-CH-ClF) | Spaltprodukte | |
|---|---|---|---|---|
| | | | % $C_2H_2ClF_3$ | % $Cl^-$ |
| 1b | 14 | - | 0,11 | 0,03 |
| 1d | 14 | DMP | 0,08 | n.g. |
| 1f | 14 | But | 0,10 | n.g. |
| 1h | 14 | DMP/But 1:1 | n.n. | n.n. |

n.n. = nicht nachweisbar (Nachweisgrenze 0,001 %) n.g. = nicht gemessen

Tabelle 3

| Beispiel | Lagerzeit (Tage) | Stabilisator (1 %, bezogen auf $CHCl_2$-$CF_3$) | Spaltprodukte | |
|---|---|---|---|---|
| | | | % $C_2H_2ClF_3$ | % $Cl^-$ |
| 2a | 14 | - | 0,19 | 0,06 |
| 2c | 14 | DMP | 0,13 | 0,04 |
| 2e | 14 | But | 0,13 | n.g. |
| 2g | 14 | DMP/But 1:1 | n.n. | n.n. |

Tabelle 4

| Beispiel | Lagerzeit (Tage) | Stabilisator (1 %, bezogen auf $CHCl_2CF_3$/ $CClF_2$-CHClF | Spaltprodukte | |
|---|---|---|---|---|
| | | | % $C_2H_2ClF_3$ | % $Cl^-$ |
| 2b | 14 | - | 0,06 | 0,05 |
| 2d | 14 | DMP | 0,02 | n.g. |
| 2f | 14 | But | 0,02 | 0,01 |
| 2h | 14 | DMP/But 1:1 | n.n. | n.n. |

n.n. = nicht nachweisbar (Nachweisgrenze 0,001 %) n.g. = nicht gemessen

Tabelle 5

| Beispiel | Lagerzeit (Tage) | Stabilisator (1 %, bezogen auf $CHCl_2$-$CF_3$) | Spaltprodukte | |
|---|---|---|---|---|
| | | | % $C_2H_2ClF_3$ | % $Cl^-$ |
| 3a | 1 | - | 0,020 | n.n. |
| | 7 | - | 0,055 | 0,0018 |
| | 14 | - | 0,549 | 0,013 |
| 3c | 14 | DMP | 0,134 | 0,036 |
| 3e | 14 | But | n.n. | 0,003 |
| 3g | 14 | DMP/But 1:1 | n.n. | n.n. |

Tabelle 6

| Beispiel | Lagerzeit (Tage) | Stabilisator (1 %, bezogen auf $CHCl_2$-$CF_3$/ $CClF_2$-$CHClF$) | Spaltprodukte | |
|---|---|---|---|---|
| | | | % $C_2H_2ClF_3$ | % $Cl^-$ |
| 3b | 1 | - | 0,034 | n.n. |
| | 7 | - | 0,067 | 0,002 |
| | 14 | - | 0,477 | 0,015 |
| 3d | 14 | DMP | 0,204 | 0,023 |
| 3f | 14 | But | n.n. | 0,001 |
| 3h | 14 | DMP/But 1:1 | n.n. | n.n. |
| n.n. = nicht nachweisbar (Nachweisgrenze 0,001 %) | | | | |
| n.g. = nicht gemessen | | | | |

Tabelle 7

| Beispiel | Lagerzeit (Tage) | Stabilisator (1 %, bezogen auf $CHCl_2$-$CF_3$) | Spaltprodukte | |
|---|---|---|---|---|
| | | | % $C_2H_2ClF_3$ | % $Cl^-$ |
| 4a | 14 | - | 0,88 | n.g. |
| 4c | 14 | DMP | 0,45 | 0,036 |
| 4e | 14 | But | 0,015 | 0,008 |
| 4g | 14 | DMP/But 1:1 | n.n. | n.n. |
| | 26 | DMP/But 1:1 | n.n. | n.n. |

Tabelle 8

| Beispiel | Lagerzeit (Tage) | Stabilisator (1 % bezogen auf $CHCl_2$-$CF_3$/ $CClF_2$-$CHClF$) | Spaltprodukte | |
|---|---|---|---|---|
| | | | % $C_2H_2ClF_3$ | % $Cl^-$ |
| 4b | 14 | - | 1,09 | n.g. |
| 4d | 14 | DMP | 0,54 | n.g. |
| 4f | 14 | But | 0,019 | n.n. |
| 4h | 14 | DMP/But 1:1 | n.n. | n.n. |
| | 26 | DMP/But 1:1 | n.n. | n.n. |
| n.n. = nicht nachweisbar (Nachweisgrenze 0,001 %) | | | | |
| n.g. = nicht gemessen | | | | |

Tabelle 9

| Beispiel | Lagerzeit (Tage) | Stabilisator-Anteile (Gew.-%) (bezogen auf $CHCl_2$-$CF_3$) | | Spaltprodukt % $C_2H_2ClF_3$ |
|---|---|---|---|---|
| | | DMP | But. | |
| 5a | 14 | 0,05 | 0,01 | 0,040 |
| 5b | 20 | 0,1 | 1,0 | 0,002 |
| 5c | 20 | 0,2 | 0,5 | n.n. |
| 5d | 20 | 0,2 | 1,0 | n.n. |
| 5e | 14 | 0,5 | 0,2 | n.n. |
| 5f | 14 | 1,0 | 0,05 | n.n. |
| 5g | 14 | 1,0 | 0,1 | n.n. |
| 5h | 14 | 1,0 | 0,5 | n.n. |
| 5k | 14 | 2,0 | 0,1 | n.n. |

**Patentansprüche**

1. Mischung, die im wesentlichen aus 2,4-Diphenyl-4-methyl-penten, 4-tert.-Butylbrenzkatechin und mindestens einem der beiden Dichlortrifluorethane $CF_3$-$CHCl_2$ und $CClF_2$-$CHClF$ besteht.

2. Mischung nach Anspruch 1, die 0,1 bis 5 Gew.-% 2,4-Diphenyl-4-methylpenten und 0,02 bis 2 Gew.-% 4-tert.-Butylbrenzkatechin enthält, bezogen auf die Menge an Dichlortrifluorethanen.

3. Mischung nach Anspruch 1, die 0,2 bis 2 Gew.-% 2,4-Diphenyl-4-methylpenten und 0,05 bis 1 Gew.-% 4-tert.-Butylbrenzkatechin enthält, bezogen auf die Menge an Dichlortrifluorethanen.

4. Verfahren zur Stabilisierung von Mischungen, die mindestens eines der Dichlortrifluorethane $CF_3$-$CHCl_2$ und $CClF_2$-$CHClF$ sowie mindestens ein Aminopolyol und/oder ein Amin enthalten, dadurch gekennzeichnet, daß man den Mischungen eine wirksame Menge 2,4-Diphenyl-4-methylpenten und 4-tert.-Butylbrenzkatechin zusetzt.

5. Verfahren nach Anspruch 4, dadurch gekennzeichnet, daß man den Mischungen 0,1 bis 5 Gew.-% 2,4-Diphenyl-4-methylpenten und 0,02 bis 2 Gew.-% 4-tert.-Butylbrenzkatechin, insbesondere 0,2 bis 2 Gew.-% 2,4-Diphenyl-4-methylpenten und 0,05 bis 1 Gew.-% 4-tert.-Butylbrenzkatechin, bezogen auf die Menge an Dichlortrifluorethanen, zusetzt.

6. Verfahren nach Anspruch 4 oder 5, dadurch gekennzeichnet, daß die Mischungen zur Herstellung von Polyurethanschaumstoffen verwendet werden.

7. Verfahren nach Anspruch 4 oder 5, dadurch gekennzeichnet, daß die Mischungen zusätzlich eine oder mehrere bei der Herstellung von Polyurethanschaum übliche sonstigen Komponenten, außer dem Polyisocyanat enthalten.

**Patentansprüche für folgende Vertragsstaaten : ES, GR**

1. Verfahren zur Stabilisierung von Mischungen, die mindestens eines der Dichlortrifluorethane $CF_3$-$CHCl_2$ und $CClF_2$-$CHClF$ sowie mindestens ein Aminopolyol und/oder ein Amin enthalten, dadurch gekennzeichnet, daß man den Mischungen eine wirksame Menge 2,4-Diphenyl-4-methylpenten und 4-tert.-Butylbrenzkatechin zusetzt.

2. Verfahren nach Anspruch 1, dadurch gekennzeichnet, daß man den Mischungen 0,1 bis 5 Gew.-% 2,4-Diphenyl-4-methylpenten und 0,02 bis 2 Gew.-% 4-tert-Butylbrenzkatechin, insbesondere 0,2 bis 2 Gew.-% 2,4-Diphenyl-4-methylpenten und 0,05 bis 1 Gew.-% 4-tert.-Butylbrenzkatechin, bezogen auf die Menge an Dichlortrifluorethanen, zusetzt.

3.  Verfahren nach Anspruch 1 oder 2, dadurch gekennzeichnet, daß die Mischungen zur Herstellung von Polyurethanschaumstoffen verwendet werden.

4.  Verfahren nach Anspruch 1 oder 2, dadurch gekennzeichnet, daß die Mischungen zusätzlich eine oder mehrere bei der Herstellung von Polyurethanschaum übliche sonstigen Komponenten, außer dem Polyisocyanat enthalten.